# FASCICULE DE BREVET EUROPEEN

(11) **EP 0 346 226 B1**
(45) Date de publication et mention de la délivrance du brevet: **17.02.1993**
(21) Numéro de dépôt: 89401577.5
(22) Date de dépôt: 07.06.1989
(51) Int. Cl.: C07C 45/00, C07C 45/51, C07C 45/42, C07C 49/697, C07C 49/755

(54) **Procédé de préparation de 4-aryl disubstitué-1-tétralones**
Verfahren zur Herstellung von 4-Aryl-disubstituierten-1-Tetralonen
Process for the preparation of 4-aryl disubstituted-1-tetralones

(30) Priorité: 08.06.1988 FR 8807641
(43) Date de publication de la demande: 13.12.1989
(73) Titulaire: SYNTHELABO, 92350 Le Plessis Robinson (FR)
(72) Inventeur: Adrian, Guy, F-69700 Givors (FR)
(74) Mandataire: Kedinger, Jean-Paul

(56) Documents cités:
- CHEMICAL ABSTRACTS, vol. 76, 1972, page 431, résumé no. 140286s, Columbus, Ohio, US; V.A. KOPTYUG et al.: "Interaction of phenols with Lewis acids. II. Aralkylation of aromatic compounds by tautomeric forms of naphthols"
- CHEMICAL ABSTRACTS, vol. 97, 1982, page 540, résumé no. 5532n, Columbus, Ohio, US; I.B. REPINSKAYA et al.: "Reaction of phenols and their derivatives with aromatic compounds in the presence of acid agents. VI. Condensation of 1-naphthol with monosubstituted benzene derivatives"

## Description

La présente invention a pour objet un nouveau procédé de préparation de 4-aryl disubstitué-1-tétralones, encore dénommées 4-aryl disubstitué-3,4 dihydro-1-(2H) naphtalènones, et plus précisément des 4-aryl disubstitué-1-tétralones répondant à la formule 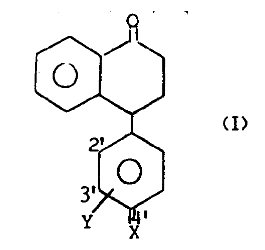
dans laquelle :
- X et Y représentent chacun un atome du chlore, et
- Y est situé en position 2′ ou 3′.

Par Chemical Abstracts, vol. 76, 1972, page 431, n^{º} 140286s et vol. 97, 1982, page 540, n^{º} 5532n, on connait un procédé de condensation du 1-naphtol avec des dérivés benzènes monosubstitués.

Les tétralones (I) sont connues pour être particulièrement utiles en tant qu'intermédiaires de synthèse pour la préparation de divers composés inhibiteurs pré-synaptiques de la sérotonine, à propriété anti-dépressive, décrits notamment dans les brevets américains Nos 4 029 731 et 4 045 488 et le brevet européen No 0 030 081.

A ce jour, les tétralones de formule (I) sont préparées par mise en oeuvre d'un procédé à 5 étapes décrit dans Organic Reaction, tome VI, pages 1 à 73 et dans le brevet européen sus-mentionné. Ce procédé connu comprend (a) la réaction du chlorure de benzoyle avec un hydrocarbure aromatique en présence de chlorure d'aluminium, ce qui conduit à des benzophénones, (b) la condensation de ces dernières, dans les conditions de la réaction de Stobbe en présence d'une base forte telle que le tertiobutylate de potassium, avec le succinate de diéthyle, (c) la décarboxylation des composés obtenus, au moyen d'une solution aqueuse d'acide bromhydrique, ce qui conduit aux acides 4,4-diaryl buténoiques, (d) l'hydrogénation sur charbon palladié de ces derniers en acides 4,4-diaryl butanoiques et (e) la cyclisation de ces derniers en 4-aryl tétralones, soit directement par l'acide fluorhydrique ou l'acide polyphosphorique, soit, après transformation en chlorures d'acide à l'aide du chlorure de thionyle, par l'action du chlorure d'aluminium dans les conditions de la réaction de Friedel-Crafts.

Ce procédé connu présente divers inconvénients. En effet, il est extrêmement long puisqu'il comporte 5 étapes qui nécessite chacune une durée de réaction relativement importante. Par ailleurs, il fait appel à des réactifs coûteux tels que le tertiobutylate de potassium et le charbon palladié, ou agressifs et donc d'emploi dangereux tels que l'acide bromhydrique, l'acide fluorhydrique et le chlorure de thionyle. En outre, en raison du grand nombre d'étapes nécessaires, le rendement global en tétralones est très faible puisque dans le meilleur des cas, il ne dépasse pas 40 % par rapport au chlorure de benzoyle.

Tous ces inconvénients constituent indéniablement des obstacles majeurs à la mise en oeuvre industrielle du procédé en cause.

Le but de la présente invention est par conséquent de remédier à ces inconvénients et pour ce faire elle propose un procédé de préparation des tétralones répondant à la formule (I) définie ci-dessus, procédé qui se caractérise en ce qu'il comprend la réaction, en présence d'un agent acide, de l'α-naphtol de formule : 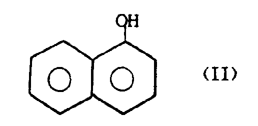
avec l'ortho-dichlorobenzène ou le méta-dichlorobenzène

Comme on pourra le constater, le procédé selon l'invention est d'une conception particulièrement simple puisqu'il ne comporte qu'une seule étape dont la mise en oeuvre est aisée, rapide, peu onéreuse et ne nécessitant pas de précaution particulière puisque n'utilisant pas de réactifs d'emploi dangereux. De plus, il permet d'obtenir les tétralones recherchées avec des rendements extrêmement élevés pouvant atteindre 80 % par rapport à l'α-naphtol ; ce procédé constitue donc une méthode de choix pour la préparation industrielle de ces tétralones.

L'agent acide mis en oeuvre dans le procédé selon l'invention, est avantageusement constitué par un acide de Lewis, notamment un tri-halogènure d'aluminium et en particulier l'acide de Lewis est choisi entre le chlorure d'aluminium, le bromure d'aluminium et leurs mélanges.

Par ailleurs, on préfère que l'ortho-dichlorobenzène ou le méta-dichlorobenzène soit utilisé en excès par rapport au composé de formule (II), les meilleurs rendements étant obtenus lorsque l'ortho-dichlorobenzène ou le méta-dichlorobenzène est utilisé à raison de 2 à 10, et en particulier de 2 à 5, équivalents molaires par rapport au composé de formule (II).

Quant à l'agent acide, il est généralement utilisé à raison de 1,5 à 3 équivalents molaires par rapport au composé de formule (II), un mode de réalisation particulièrement préféré consistant à utiliser, en tant qu'agent acide environ 2 moles de chlorure d'aluminium par mole du compose de formule (II).

La réaction du composé de formule (II) avec l'ortho-dichlorobenzène ou le méta-dichlorobenzène est de préférence effectuée à chaud, par exemple entre 40 et 100° C et notamment entre 50 et 70° C.

L'ortho-dichlorobenzène ou le méta-dichlorobenzène, utilisé en excès, peut jouer le rôle de solvant et la réaction peut alors par exemple être effectuée indifféremment par introduction de l'agent acide dans une suspension de l'α-naphtol (II) dans ledit dichlorobenzène ou par addition de l'α-naphtol dans une suspension convenablement agitée de l'agent acide dans le dichlorobenzène. Toutefois, rien ne s'oppose à effectuer la réaction dans un solvant organique qui sera choisi de préférence parmi les hydrocarbures aliphatiques polyhalogénés et leurs mélanges, ce solvant pouvant par exemple être constitué par le dichloro-1,2 éthane.

La réaction est généralement complète après 1 à 3 heures, cette durée étant toutefois variable notamment suivant la température de réaction et la quantité d'agent acide et de dichlorobenzène mis en jeu par rapport au composé (II).

Conformément à la présente invention, le produit de réaction du composé de formule (II) avec l'ortho-dichlorobenzène ou le méta-dichlorobenzène, est ensuite soumis à une hydrolyse, par de l'eau ou de l'eau acidulée par exemple pare de l'acide chlorhydrique, notamment à une température inférieure à 40°C.

Les tétralones recherchées peuvent être isolées et purifiées par mise en oeuvre des techniques classiques bien connues de l'Homme de métier et notamment par élimination par distillation de l'ortho-dichlorobenzène ou le méta-dichlorobenzène en excès, ce dernier étant avantageusement recyclé, suivie de l'isolement de la tétralone, soit par distillation soit par cristallisation dans un solvant approprié.

Les composés de formule (I) obtenus par mise en oeuvre de l'ortho-dichlorobenzène ou le méta-dichlorobenzène, permettant la préparation de composés finals à propriété anti-dépressive particulièrement intéressants.

L'invention est illustrée ci-après par un certain nombre d'exemples.

### Exemple 1 :

préparation de la 4-(3′,4′-dichlorophényl)-3,4-dihydro-1-(2H)-naphtalènone

Dans un réacteur de 1 litre équipé d'un dispositif d'agitation à ancre tournante, on place 144,2 g (1 mole) d'α-naphtol et 500 ml d'ortho dichlorobenzène. On ajoute, en 20 mn et par portions, 280 g (2,1 moles) de chlorure d'aluminium anhydre. La température s'élève de 20 à 55° C et la suspension jaune épaisse se dissout progressivement pour donner une solution verte.

On chauffe 3 heures à 65° C, puis coule le milieu réactionnel sur 1500 ml d'eau. La phase organique inférieure est séparée par décantation et la phase aqueuse réextraite par 300 ml d'orthodichlorobenzène.

La phase organique est concentrée sous vide à une température comprise entre 60 et 80 ° C sous une pression 2 à 5 torrs et l'orthodichlorobenzène ainsi récupéré, soit 650 ml, peut être recyclé. Le concentrat (300 g environ) est repris par 400 ml de méthyléthylcétone et 2 g de charbon décolorant à 60° C, puis filtré et dilué par 250 ml de méthanol. Cette solution est refroidie pendant 4 heures à 0° C. Le solide beige clair formé est filtré et séché ce qui permet d'obtenir 177,8 g du composé attendu.
. Rendement 61 % par rapport à l'α-naphtol de départ
. Point de fusion : 104-105°C
. Pureté CGL : 98-99 %

Une pureté CGL supérieure à 99,5 % peut être obtenue par recristallisation dans un mélange de 2 parties de méthyléthylcétone et de 2,5 parties de méthanol.

### Exemple 2 :

préparation de la 4-(3′,4′-dichlorophényl)-3,4-dihydro-1-(2H)-naphtalènone

Dans une suspension de 280 g (2,1 moles) de chlorure d'aluminium anhydre dans 500 ml d'orthodichlorobenzène, on introduit en 20 mn 144,2 g (1 mole) d'α-naphtol. Le milieu est chauffé pendant 3 heures à 65° C, puis hydrolysé à une température inférieure à 40° C par 1 litre d'eau.

On ajoute 700 ml d'éther di-isopropylique, décante et sépare la phase organique supérieure que l'on maintient pendant 4 heures entre - 5 et - 10°C.

Le précipité formé est essoré et séché pour obtenir 148 g du composé attendu.
. Rendement : 50,9 % par rapport à l'α-naphtol
. Point de fusion : 105°C
. Pureté CGL : 99 %

### Exemple 3 :

préparation de la 4-(3′,4′-dichlorophényl)-3,4-dihydro-1-(2H)-naphtalènone

Dans une solution de 100 g (0,374 mole) de bromure d'aluminium anhydre dans 130 ml d'orthodichlorobenzène, on introduit 28 g (0,194 mole) d'α-naphtol. La solution verte obtenue est chauffée pendant 1 heure à 65°C puis hydrolysée par 400 ml d'eau à 40°C.

Après décantation, on sépare la phase organique que l'on concentre sous vide. Après cristallisation du résidu dans 140 ml de méthanol à 0 ° C pendant 2 heures, on obtient, après filtration et séchage, 45 g du composé attendu.
. Rendement : 79,6 % par rapport à l'α-naphtol
. Point de fusion : 104° C
. Pureté CGL : 98,5 %

### Exemple 4 :

préparation de la 4-(2′,4′-dichlorophényl)-3,4-dihydro-1-(2H)-naphtalènone

Dans une suspension de 60 g (0,45 mole) de chlorure d'aluminium dans 100 ml de métadichlorobenzène, on introduit 28,8 g (0,2 mole) d'α-naphtol en 10 mn ; la température s'élève de 20 à 45° C. Le milieu est chauffé pendant 1 h 30 à 65°C, puis hydrolysé par 400 ml d'eau. Après décantation, la phase organique inférieure est séparée et concentrée sous un vide de 2 à 5 torrs à 80° C. Le résidu est dissout dans 100 ml d'isopropanol et la solution résultante refroidie à 0° C pendant 2 heures. Le solide beige clair formé est filtré et séché pour obtenir 41,5 g du composé attendu.
. Rendement : 71 % par rapport à l'α-naphtol
. Point de fusion : 123-124°C
. Pureté CGL : 99 %
. spectre RMN (CDCl₃), δ (ppm) = 2,20-2,80 (m, 4H) ; 4,80 (t, 1H) ; 6,80-8,10 (m, 7H)

## Revendications

1. Procédé de préparation de 4-aryl disubstitué-1-tétralones de formule 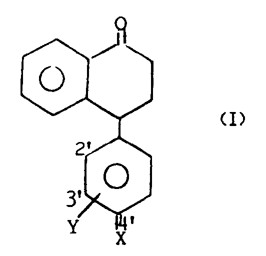 dans laquelle :
- X et Y représentent chacun un atome de chlore, et
- Y est situé en position 2′ ou 3′,
caractérisé en ce qu'il comprend la réaction, en présence d'un agent acide, de l'α-naphtol de formule : 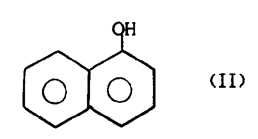 avec l'ortho-dichlorobenzène ou le méta-dichlorobenzène.

2. Procédé selon la revendication 1, caractérisé en ce qu'il comprend en outre l'hydrolyse du produit de réaction du composé de formule (II) avec l'ortho-dichlorobenzène ou le méta-dichlorobenzène.

3. Procédé selon la revendication 2, caractérisé en ce que l'hydrolyse est une hydrolyse acide.

4. Procédé selon l'une des revendications précédentes, caractérisé en ce que l'agent acide est un acide de Lewis.

5. Procédé selon la revendication 4, caractérisé en ce que l'acide de Lewis est un tri-halogènure d'aluminium.

6. Procédé selon la revendication 4, caractérisé en ce que l'acide de Lewis est choisi entre le chlorure d'aluminium, le bromure d'aluminium et leurs mélanges.

7. Procédé selon l'une revendications précédentes, caractérisé en ce que l'ortho-dichlorobenzène ou le méta-dichlorobenzène est utilisé en excès par rapport au composé de formule (II).

8. Procédé selon la revendication 7, caractérisé en ce que l'ortho-dichlorobenzène ou le méta-dichlorobenzène est utilisé à raison de 2 à 5 équivalents molaires par rapport au composé de formule (II).

9. Procédé selon l'une des revendications précédentes, caractérisé en ce que l'agent est utilisé à raison de 1,5 à 3 équivalents molaires par rapport à l'ortho-dichlorobenzène ou le méta-dichlorobenzène.

10. Procédé selon la revendication 9, caractérisé en ce que l'agent acide est constitué par le chlorure d'aluminium utilisé à raison de 2,1 moles par mole du composé de formule (II).

11. Procédé selon l'une des revendications précédentes, caractérisé en ce que la réaction du composé de formule (II) avec l'ortho-dichlorobenzène ou le méta-dichlorobenzène est effectuée entre 50 et 70° C.

12. Procédé selon l'une des revendications précédentes, caractérisé en ce que la réaction du composé de formule (II) avec l'ortho-dichlorobenzène ou le méta-dichlorobenzène est effectuée dans un solvant organique choisi parmi les hydrocarbures aliphatiques polyhalogénés et leurs mélanges.

## Patentansprüche

1. Verfahren zur Herstellung von 4-Aryl-disubstituierten-1-Tetralonen der Formel 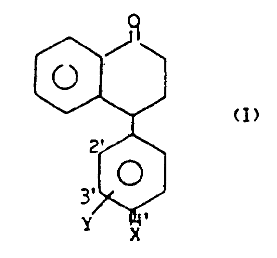 in welcher:
- X und Y jeweils ein Chloratom bilden und
- Y in der Position 2′ oder 3′ liegt,
dadurch gekennzeichnet, daß es in Anwesenheit eines säurehaltigen Mittels die Reaktion des α-Naphtols der Formel 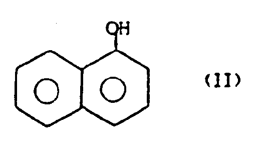 mit Orthodichlorbenzol oder Metadichlorbenzol umfaßt.

2. Verfahren nach Anspruch 1, dadurch gekennzeichnet, daß es außerdem die Hydrolyse des Reaktionsprodukts der Verbindung der Formel (II) mit Orthodichlorbenzol oder Metadichlorbenzol umfaßt.

3. Verfahren nach Anspruch 2, dadurch gekennzeichnet, daß die Hydrolyse eine Säurehydrolyse ist.

4. Verfahren nach einem der vorhergehenden Ansprüche, dadurch gekennzeichnet, daß das säurehaltige Mittel eine Lewis-Säure ist.

5. Verfahren nach Anspruch 4, dadurch gekennzeichnet, daß die Lewis-Säure ein Aluminium-Trihalogenid ist.

6. Verfahren nach Anspruch 4, dadurch gekennzeichnet, daß die Lewis-Säure unter Aluminiumchlorid, Aluminiumbromid und ihren Mischungen ausgewählt ist.

7. Verfahren nach einem der vorhergehenden Ansprüche, dadurch gekennzeichnet, daß Orthodichlorbenzol oder Metadichlorbenzol in Bezug auf die Verbindung der Formel (II) im Überschuß verwendet wird.

8. Verfahren nach Anspruch 7, dadurch gekennzeichnet, daß Orthodichlorbenzol oder Metadichlorbenzol im Verhältnis von 2 bis 5 Moläquivalenten in Bezug auf die Verbindung der Formel (II) verwendet wird.

9. Verfahren nach einem der vorhergehenden Ansprüche, dadurch gekennzeichnet, daß das säurehaltige Mittel im Verhältnis von 1.5 bis 3 Moläquivalenten in Bezug auf Orthodichlorbenzol oder Metadichlorbenzol verwendet wird.

10. Verfahren nach Anspruch 9, dadurch gekennzeichnet, daß das säurehaltige Mittel Aluminiumchlorid ist und im Verhältnis von 2.1 Mols je Mol der Verbindung der Formel (II) verwendet wird.

11. Verfahren nach einem der vorhergehenden Ansprüche, dadurch gekennzeichnet, daß die Reaktion der Verbindung der Formel (II) mit Orthodichlorbenzol oder Metadichlorbenzol bei 50 bis 70°C bewirkt wird.

12. Verfahren nach einem der vorhergehenden Ansprüche, dadurch gekennzeichnet, daß die Reaktion der Verbindung der Formel (II) mit Orthodichlorbenzol oder Metadichlorbenzol in einem organischen Lösungsmittel bewirkt wird, welches unter den aliphatischen polyhalogenisierten Kohlenwasserstoffen und ihren Mischungen ausgewählt ist.

## Claims

1. Method of preparing 4-disubstituted phenyl-1-tetralones of formula 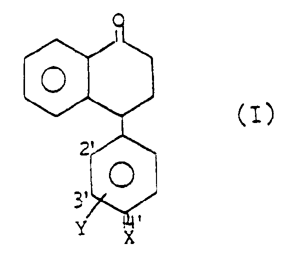 in which :
- X and Y each represents a chlorine atom, and
- Y is situated in position 2′ or 3′,
characterized in that it comprises the reaction, in the presence of an acid agent, of α-naphthol of formula : 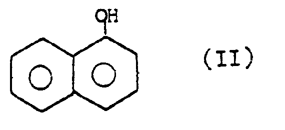 with ortho-dichlorobenzene or meta-dichlorobenzene.

2. Method according to claim 1, characterized in that it further comprises the hydrolysis of the reaction product of the compound of formula (II) with ortho-dichlorobenzene or meta-dichlorobenzene.

3. Method according to claim 2, characterized in that the hydrolysis is an acid hydrolysis.

4. Method according to claim 1 or 2, characterized in that the acid agent is a Lewis acid.

5. Method according to claim 4, characterized in that the Lewis acid is an aluminium tri-halide.

6. Method according to claim 4, characterized in that the Lewis acid is chosen from aluminium chloride, aluminium bromide and mixtures thereof.

7. Method according to the preceding claims, characterized in that ortho-dichlorobenzene or meta-dichlorobenzene is used in excess with respect to the compound of formula (II).

8. Method according to claim 7, characterized in that ortho-dichlorobenzene or meta-dichlorobenzene is used at the ratio of 2 to 5 molar equivalents with respect to the compound of formula (II).

9. Method according to one of the preceding claims, characterized in that the acid agent is used at the ratio of 1,5 to 3 molar equivalents with respect to ortho-dichlorobenzene or meta-dichlorobenzene.

10. Method according to claim 9, characterized in that the acid agent is formed by aluminium chloride used at the ratio of 2,1 moles per mole of the compound of formula (II).

11. Method according to one of the preceding claims, characterized in that the reaction of the compound of formula (II) with the ortho-dichlorobenzene or meta-dichlorobenzene is carried out between 50 and 70°C.

12. Method according to one of the preceding claims, characterized in that the reaction of the compound of formula (II) with the ortho-dichlorobenzene or meta-dichlorobenzene is effected in an organic solvent chosen from the polyhalogenated aliphatic hydrocarbons and mixtures thereof.
